# EUROPÄISCHE PATENTANMELDUNG

(11) **EP 1 283 491 A2**
(43) Veröffentlichungstag der Anmeldung: **12.02.2003**
(21) Anmeldenummer: 02016800.1
(22) Anmeldetag: 26.07.2002
(51) Int. Cl.: G06F 19/00

(54) **Qualitätskontrollsystem in Disease Management Services zur Kontrolle der Therapietreue**

(30) Priorität: 07.08.2001 DE 10138708
(71) Anmelder: SIEMENS AKTIENGESELLSCHAFT, 80333 München (DE)
(72) Erfinder: Abraham-Fuchs, Klaus, 91058 Erlangen (DE); Bieger, Johannes, Dr., 80638 München (DE); Rumpel, Eva, Dr., 91052 Erlangen (DE); Schmidt, Kai-Uwe, Dr., 91052 Erlangen (DE); Tietze, Daniel, 91080 Spardorf (DE)

(57) **Zusammenfassung**

Qualitätskontrollsystem in Disease Management Services mit Aufklärung, Weiterbildung und Motivation von an chronischen Volkskrankheiten, wie Diabetes, Asthma oder Hypertonie leidenden Patienten, gegebenenfalls mit Telemonitoring kritischer Körperwerte und daraus resultierende Früherkennung bzw. Vermeidung von Risikosituationen, wobei eine automatische Zugangsüberwachungseinrichtung vorgesehen, die den Eingang aller Mitteilungen und Informationen an die Patienten überwacht und gegebenenfalls eine Kontrollstelle zur Behebung von Zugangsproblemen informiert.

## Beschreibung

Die Erfindung bezieht sich auf ein Qualitätskontrollsystem in Disease Management Services mit Aufklärung, Weiterbildung und Motivation von an chronischen Volkskrankheiten, wie Diabetes, Asthma oder Hypertonie leidenden Patienten, gegebenenfalls mit Telemonitoring kritischer Körperwerte und daraus resultierende Früherkennung bzw. Vermeidung von Risikosituationen.

Disease Management Service Provider (DMSP) sind als medizinische Dienstleister weltweit eine Wachstumsbranche. Ihr Unternehmenskonzept beruht vornehmlich auf Patientenaufklärung, - weiterbildung und -motivation, kombiniert mit Telemonitoring kritischer Körperwerte und daraus frühzeitige Erkennung bzw. Vermeidung von Risikosituationen. Disease Management Services betreuen typischerweise Patienten, die an einer chronischen Volkskrankheit leiden, wie zum Beispiel Diabetes, Asthma oder Hypertonie, womit große Patientenzahlen mit einem weitgehend standardisierten Behandlungsplan über lange Zeiträume (typischerweise Monate oder Jahre) betreut werden. Dies führt zu einer deutlich gesteigerten Kosteneffizienz gegenüber traditioneller Patientenversorgung. Diese Kosteneffizienz wird dabei unter anderem durch eine möglichst weitgehende Automatisierung der Patientenbetreuung erreicht, indem zum Beispiel in festgelegten Intervallen automatisch Patientenschulungsmaterial verschickt, Beratungsanrufe durchgeführt, oder Messwerte, wie zum Beispiel der Blutdruck vom Patienten in digitaler Form an ein Kontrollzentrum gesendet, dort automatisch bewertet und bei Über- bzw. Unterschreitung von Grenzwerten ein Fax oder ein e-mail an den betreuenden Arzt mit einer entsprechenden Behandlungsempfehlung versendet wird.

In der Regel werden die Kosten für diese Form der Patientenbetreuung durch die Krankenkassen übernommen. Letztere fordern zunehmend von den DMSP einen Nachweis über die langfristige Kosteneffizienz einer solchen Intervention ein. Da es sich bei den zu betreuenden Krankheitsbildern im wesentlichen um solche handelt, bei denen die Patienten gewohnte aber ungesunde Handlungsweisen verändern müssen (zum Beispiel Rauchen einstellen, Ernährung verändern etc.), lässt sich der gewünschte Erfolg nur durch Einsicht und Verständnis des Patienten für seine Situation realisieren. Daher kommt der Patienten-Edukation, also der Durcharbeitung des entsprechenden Schulungsmaterials und der entsprechenden inhaltlichen Durchdringung eine besondere Bedeutung zu.

Um also die sehr wichtige Frage klären zu können, ob der zu betreuende Patient das ihm zugewiesene Schulungsmaterial a) erhalten und b) gemäß den Vorgaben bearbeitet und auch verstanden hat, bleibt bisher dem Service Provider nichts anderes übrig, als den Patienten telefonisch einem Assessment zu unterziehen, welches sich, je nach Art des Patienten, sehr aufwändig und langwierig gestalten kann.

Andererseits kann der DMSP nicht auf dieses Vorgehen verzichten, da es zum einen eine Form der Betreuungsdokumentation gegenüber Kassen darstellt und zum anderen Krankenkassen zunehmend erfolgsabhängig abrechnen wollen und damit die Service Provider eine besonders große Motivation haben, dass der Patient das Schulungsmaterial verinnerlicht und umsetzt.

Der Erfindung liegt daher die Aufgabe zugrunde, ein Qualitätskontrollsystem der eingangs genannten Art so auszugestalten, dass eine automatisierte Kontrolle des Wissensstandes des Patienten im Rahmen des individuellen Schulungsprogramms möglich ist.

Zur Lösung dieser Aufgabe ist erfindungsgemäß zunächst eine automatische Zustellungsüberwachungseinrichtung vorgesehen, die den Eingang aller Mitteilungen und Informationen an die Patienten überwacht und gegebenenfalls eine Kontrollstelle zur Behebung von Zugangsproblemen informiert. Im Falle der e-mail-Versendung der Unterlagen kann dies durch eine entsprechenden e-mail-Antwort und im Falle der Online-Version durch Aufzeichnen der Aktivität des jeweiligen Patienten erfolgen (zum Beispiel durch Log-Files).

In einer weiteren Stufe wird der Patient einem Verständnistest unterzogen, dessen Eingang beim Patienten in der Versendezentrale des Disease Management Service Providers automatisiert überwacht und dessen Durchführungsergebnisse vorzugsweise vollautomatisch ausgewertet werden, wobei gemäß einem weiteren Merkmal der Erfindung durch die Auswerteeinheit automatische Warnhinweise an den Arzt, den Patienten oder die jeweilige Krankenkasse generierbar sind.

Durch diese Verständnistests lässt sich feststellen, ob der Patient die Lektionen und Hinweise und Informationen ausreichend verstanden hat, wobei diese Verständnistests auch zur Auffrischung zurückliegender Lektionen verwendet werden können. Im Falle einer Überforderung oder bei permanentem Desinteresse des Patienten kann dieser dann einbestellt werden und die entsprechenden Maßnahmen mit diesem besprochen werden. Gegebenenfalls kann auch ein Abbruch der Behandlung unter Kontaktaufnahme mit der Krankenkasse die einzig sinnvolle Konsequenz bei dauerhaften Fehlschlägen einer koordinierten Zusammenarbeit die Folge sein.

Die Tests können zum Beispiel Fragebögen (Multiple Choice oder vollständige Antworten) oder auch interaktive "Computerspiele" sein, in denen typische Szenarios durchgespielt werden, wobei der Patient an geeigneter Stelle eingreifen muss.

Die Ausgestaltung des erfindungsgemäßen Qualitätskontrollsystems kann dabei so getroffen sein, dass die positiv beurteilten Tests eine automatische Freischaltung für weitere Tests und/oder aufbauende Trainingseinheiten bewirken.

Dabei hat es sich als besonders zweckmäßig erwiesen, wenn die Tests nicht starr aufgebaut sind, sondern mithilfe von Software-Expertenregeln im Hintergrund flexibel gestaltet werden, sodass die Testfragen individualisiert auf den Patienten angepasst sind, beispielsweise abhängig von Alter, Bildungsgrad, anderen Erkrankungen, der Krankheitsgeschichte usw., wobei die hierfür notwendige Information das Expertensystem aus einer "elektronischen Patientenakte" des DMS-Providers entnehmen kann.

In Weiterbildung der Erfindung kann der Ablauf der Testfragen auch dynamisch verändert werden, zum Beispiel derart, dass jeweils beim Eingang einer falschen Antwort eine andere Art der Fragestellung folgt, um mögliche Missverständnisse des Patienten zu erkennen und zu beheben.

Letztendlich sind in einem intelligent gestalteten Lernmaterial Lernlektion und Test des Lernerfolgs miteinander verwoben unter Einbeziehung der Funktionalität der vorstehenden Testvariationen.

Die Testergebnisse können auch dazu genutzt werden, um folgenden Zusatznutzen zu erzielen: Ein dynamisch aufgebauter Behandlungsplan des Patienten enthält Handlungsanweisungen an den behandelnden Arzt und den Patienten, zusammen mit Zeitpunkten der vorgesehenen Ausführung, bzw. logische Regeln, die zu einer Ausführung der Handlungsanweisungen führen. Dies kann beispielsweise dergestaltet sein, dass gefordert wird, dass die Behandlungsregime A eingehalten werden soll, solange das Gewicht des Patienten kleiner 80 kg ist, dass aber bei einem Gewicht größer 80 kg eine Behandlung nach Regime B erfolgen soll. Die Ergebnisse der Tests des Lernerfolgs können in Form von quantifizierten Noten oder Scores zur automatischen Steuerung eines solchen Behandlungsplans herangezogen werden.

Nähere Details und Besonderheiten des erfindungsgemäßen Qualitätskontrollsystems ergeben sich aus den beigefügten Zeichnungen. Dabei zeigen:

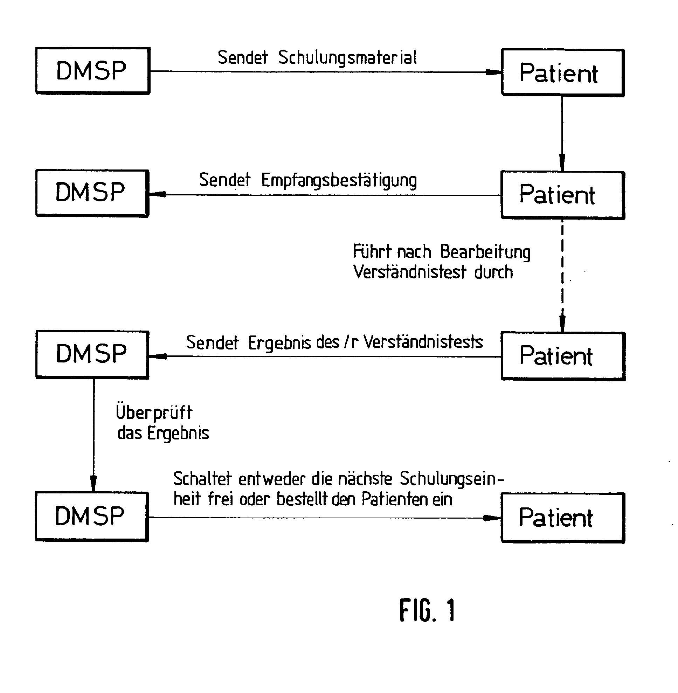

- Fig. 1: ein schematisches Ablaufdiagramm der Beziehungen Zwischen Disease Management Service Provider (DMSP) und dem Patienten und

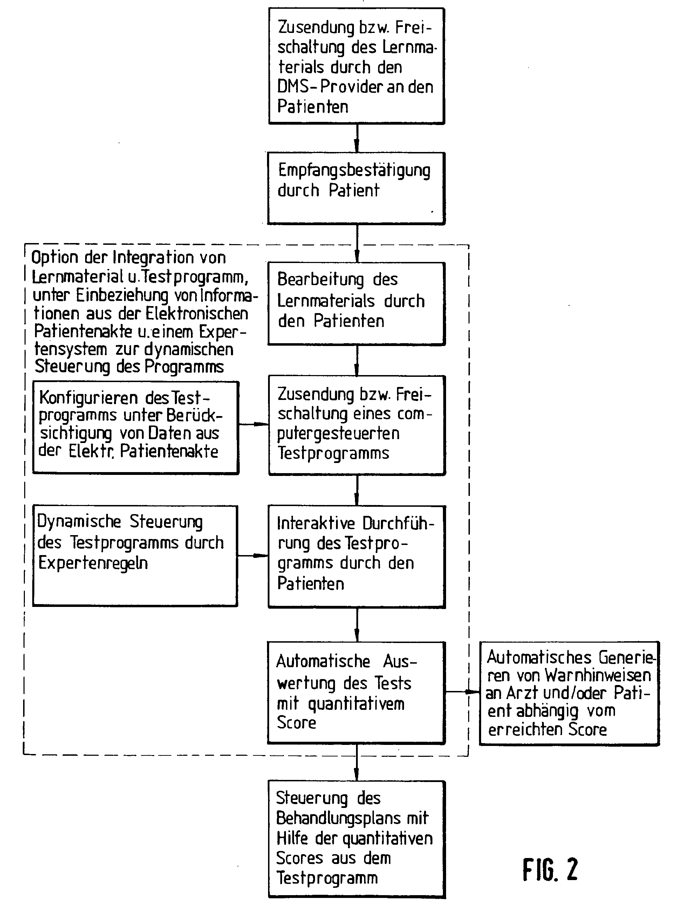

- Fig. 2: ein detailliertes Diagramm eines Behandlungsablaufs mithilfe des erfindungsgemäßen Qualitätskontrollsystems.

## Patentansprüche

1. Qualitätskontrollsystem in Disease Management Services mit Aufklärung, Weiterbildung und Motivation von an chronischen Volkskrankheiten, wie Diabetes, Asthma oder Hypertonie leidenden Patienten, gegebenenfalls mit Telemonitoring kritischer Körperwerte und daraus resultierende Früherkennung bzw. Vermeidung von Risikosituationen **dadurch gekennzeichnet, dass** eine automatische Zustellungsüberwachungseinrichtung vorgesehen ist, die den Eingang aller Mitteilungen und Informationen an die Patienten überwacht und gegebenenfalls eine Kontrollstelle zur Behebung von Zustellungsproblemen informiert.

2. Qualitätskontrollsystem nach Anspruch 1, **dadurch gekennzeichnet, dass** jeder Mitteilungseingang vom Patienten an die Versendezentrale bestätigt wird.

3. Qualitätskontrollsystem nach Anspruch 1 oder 2, **dadurch gekennzeichnet, dass** die Patienteninformation Verständnistests beinhaltet, deren Eingang beim Patienten in der Versendezentrale automatisiert überwacht und deren Ergebnisse nach Ausübung durch den Patienten durch eine Auswerteeinheit vorzugsweise vollautomatisch ausgewertet wird.

4. Qualitätskontrollsystem nach Anspruch 3, **dadurch gekennzeichnet, dass** durch die Auswerteeinheit automatische Warnhinweise an den Arzt und /oder den Patienten oder die jeweilige Krankenkasse generierbar sind.

5. Qualitätskontrollsystem nach Anspruch 3 oder 4, **dadurch gekennzeichnet, dass** die Tests Fragebögen und/oder interaktive Computerspiele umfassen.

6. Qualitätskontrollsystem nach einem der Ansprüche 3 bis 5, **dadurch gekennzeichnet, dass** die positiv beurteilten Tests eine automatische Freischaltung für weitere Tests und/oder aufbauende Trainingseinheiten bewirken.

7. Qualitätskontrollsystem nach einem der Ansprüche 3 bis 6, **dadurch gekennzeichnet, dass** die Tests mithilfe von Software-Expertenregeln flexibel gestaltet sind, insbesondere an die besonderen Umstände des einzelnen Patienten angepasst sind.

8. Qualitätskontrollsystem nach Anspruch 7, **dadurch gekennzeichnet, dass** der Ablauf der Testfragen innerhalb eines Tests bei Eingang falscher Antworten dynamisch verändert werden.

9. Qualitätskontrollsystem nach einem der Ansprüche 3 bis 8, **dadurch gekennzeichnet , dass** mithilfe der in Form von quantifizierten Noten oder Scores ausgewerteten Testergebnisse ein dynamisch aufgebauter Behandlungsplan für den Patienten erstellt wird.

10. Qualitätskontrollsystem nach einem der Ansprüche 1 bis 9, **gekennzeichnet durch** ein automatisches Generieren von Warnhinweisen oder Alarmen an Arzt und/oder Patienten abhängig vom erreichten Score bei Ausführung des Tests.
